# EUROPÄISCHE PATENTSCHRIFT

(11) **EP 0 923 389 B1**
(45) Veröffentlichungstag und Bekanntmachung des Hinweises auf die Patenterteilung: **09.04.2003**
(21) Anmeldenummer: 98943732.2
(22) Anmeldetag: 17.07.1998
(51) Int. Cl.: A61L 27/00

(54) **IN VIVO ABBAUBARES METALLISCHES IMPLANTAT**
METALLIC IMPLANT WHICH IS DEGRADABLE IN VIVO
IMPLANT METALLIQUE DEGRADABLE IN VIVO

(30) Priorität: 18.07.1997 DE 19731021
(43) Veröffentlichungstag der Anmeldung: 23.06.1999
(60) Teilanmeldung: 02019905.5
(73) Patentinhaber: Meyer, Jörg, 20357 Hamburg (DE)
(72) Erfinder: HEUBLEIN, Bernd, D-30627 Hannover (DE); HAUSDORF, Gerd, D-30938 Burgwedel (DE)
(74) Vertreter: Lenzing, Andreas, Dr.
(86) Internationale Anmeldenummer: EP9804415
(87) Internationale Veröffentlichungsnummer: WO99003515

(56) Entgegenhaltungen:
- US-A- 3 687 135

## Beschreibung

Die vorliegende Erfindung betrifft Implantate aus metallischen Werkstoffen zum Einsatz in dem menschlichen oder tierischen Körper.

Derartige Implantate sind grundsätzlich seit langem bekannt. Die ersten Implantate wurden zu orthopädischen Zwecken entwickelt, beispielsweise Schrauben und Nägel zum Fixieren von Knochenbrüchen. Diese bestanden zunächst aus relativ einfachen Eisenlegierungen, die unter in vivo-Bedingungen zu Korrosion neigten. Die Korrosion führte dazu, daß in unmittelbarer Nähe des Knochens Metalle als Ionen freigesetzt wurden, die einen unerwünschten Anreiz für das Wachstum des Knochengewebes gegeben haben. Der Knochen ist stärker gewachsen, als es eigentlich erwünscht und erforderlich ist. Hierdurch wurde das gesunde Knochenmaterial geschädigt.

Aus diesem Grund ist man bestrebt, metallische Implantate grundsätzlich aus möglichst korrosionsbeständigen Materialien zu fertigen. Hier sind derzeit hauptsächlich korrosionsbeständige Edelstähle, Tantal und Titan im Gebrauch. Diese Implantate bleiben nach der Implantierung als Fremdkörper präsent und werden als solche vom Organismus erkannt. Sie sind nur durch eine zweite Operation zu entfernen.

Außerdem sind metallische Implantate im Bereich der Gefäßchirurgie und der Kardiologie, Angiologie und Radiologie bekannt. Diese Implantate umfassen zum Beispiel endoluminale und Gefäßstützen (Stents) zur Behandlung von Läsionen. Diese Stützen dienen z. B. zur Aufweitung und Lumenerhaltung von verengten Gefäßen, indem sie vom Gefäßlumen ausgehend mit einem Ballonkatheter (balloon expandable) oder selbstexpandierend (self expanding) das Gefäßlumen auf einem entsprechend optimalen Innendurchmesser halten. Das Implantat ist an sich nur so lange erforderlich, bis das erkrankte Gefäß durch biologische Reparaturvorgänge aus eigener Kraft den erforderlichen Durchmesser dauerhaft halten kann. Dies ist im allgemeinen etwa 4 Wochen nach Implantation der Fall.

Der dauerhafte Verbleib eines metallischen Implantats ist jedoch mit einigen Nachteilen verbunden. Das Implantat führt als Fremdkörper zu lokalen und eventuell auch systemischen Reaktionen. Zusätzlich wird die Selbstregulation des betroffenen Gefäßsegments behindert. Die ständige (pulsatile) Belastung des Metalls kann zu Ermüdungsbrüchen führen, was bei großlumigen Implantaten (z. B. Verschlußsystemen wie Schirmchen) zu neuen medizinischen Problemen führen kann. Gefäßstützen in kleineren Lumina (2,5 - 6 mm) erzeugen in etwa 20% eine erneute Stenosierung (sogenannte In-Stent-Stenose), was bei der hohen Zahl der Implantate kumulativ zu einer zusätzlichen medizinischen und ökonomischen Belastung führt. In einigen Gefäßregionen (z. B. extrakranielle Gefäße, Beinarterien) kann die metallische Struktur durch Krafteinwirkung von außen dauerhaft verformt werden mit den Folgen einer erneuten Gefäßobstruktion bzw. eines induzierten Gefäßverschlusses. Jedes Dauerimplantat ist zusätzlich mit Problemen insbesondere für jüngere Patienten deshalb verbunden, weil ein Verbleiben für Jahrzehnte unausweichlich ist.

Vollkommen biologisch abbaubare Implantate sind bislang nur aus Kunststoffmaterialien bekannt, beispielsweise aus der DE 2502884 C2. Dort wird eine Beschichtung eines orthopädischen Implantats mit Polymethylmethacrylat offenbart, das biodegradabel ist. Andere Kunststoffmaterialien umfassen Polylactid- und Polyglycolsäureester. Außerdem ist aus der EP 0006544 B1 ein biodegradables Keramikmaterial auf Basis von Calciumphosphat bekannt, das ebenfalls zur Beschichtung von metallischen Implantaten dient.

Schließlich ist aus der WO 81/02668 ein orthopädisches Implantat bekannt, das einen korrosionsbeständigen metallischen Grundkörper sowie eine biologisch abbaubare, metallische Zwischenschicht für den Kontaktbereich zum Knochen ausweist. Diese Zwischenschicht bildet zusammen mit dem Grundkörper eine elektrochemische Zelle und erzeugt eine elektrische Spannung, die das Knochenwachstum fördert. Gleichzeitig wird die Oberflächenschicht, die beispielsweise aus Silberlegierungen bestehen kann, abgebaut. Dies führt zu dem angestrebten Effekt, daß das Knochenwachstum so lange positiv beeinflußt wird, wie es erforderlich ist und dann nach vollständigem Abbau der Oberflächenbeschichtung der elektrische Reiz nachläßt.

Bisher bekannte biodegradable Substanzen auf Polymerbasis werden in der Gefäßchirurgie verwendet. Ihre mechanischen Eigenschaften einerseits und die nachfolgende Fremdkörperreaktion während der Biodegradation andererseits führen dazu, daß sie als alleiniges Material für eine Implantation ungeeignet sind. Metallische Werkstoffe/Legierungen besitzen günstige mechanische Eigenschaften (Elastizität, Verformbarkeit, Stabilität) bei geringerer Masse, was für die Applikation durch dünnlumige Führungssysteme bei transkutanem Vorgehen eine wichtige Voraussetzung darstellt.

Es ist daher Aufgabe der vorliegenden Erfindung, Implantate aus biodegradablem Material zur Verfügung zu stellen, die zugleich vorteilhafte mechanische Eigenschaften aufweisen.

Diese Aufgabe wird durch Implantate mit den Merkmalen der Ansprüche 1, 2 und 3 gelöst.

Weil das medizinische Implantat aus einem metallischen Werkstoff gefertigt ist, der durch Korrosion in vivo abbaubar ist, liegen primär die mechanischen Vorteile metallischer Werkstoffe vor. Der korrosive Abbau des Implantats innerhalb einer durch Materialwahl einstellbaren Zeitskala verhindert andererseits, daß die negativen Langzeiteffekte des metallischen Fremdkörpers eintreten. Dabei ist es biologisch vorteilhaft, wenn der Werkstoff Reineisen gegebenenfalls mit einem Anteil von bis zu 7% Kohlenstoff oder eine Legierung ist, deren Hauptbestandteil Eisen ist. Derzeit wird als Hauptbestandteil Eisen bevorzugt.

Die biologischen, mechanischen und chemischen Eigenschaften der Werkstoffe sind positiv beeinflußbar, wenn als Nebenbestandteil Mangan, Kobalt, Nickel, Chrom, Kupfer, Cadmium, Blei, Zinn, Thorium, Zirkonium, Silber, Gold, Palladium, Platin, Rhenium, Silicium, Calcium, Lithium, Aluminium, Zink, Eisen, Kohlenstoff oder Schwefel vorgesehen ist. Als Material wird insgesamt derzeit eine Eisenlegierung mit einem geringen Anteil an Aluminium, Magnesium, Nickel und/oder Zink bevorzugt.

Das medizinische Implantat wird in mehreren Grundvarianten ausgeführt. Für eine Gefäßstütze ist als Grundkörper ein rohrförmiger Aufbau mit zusätzlicher Bearbeitung vorgesehen. Als Verschlußsystem (z. B. Ductus Botalli, angeborene und erworbene Septumdefekte, arteriovenöse Shuntverbindungen) sind passiv und/oder aktiv entfaltbare Schirmformen, Spiralen oder komplexe Körper vorteilhaft. Die Erfindung ist auch anwendbar bei Okkludern als Verschlußsysteme für Hohlraumverbindungen, Gefäße oder Gangsysteme.

Es ist außerdem vorteilhaft, das Implantat als Befestigungs- oder Stützvorrichtung für die temporäre Fixierung von Gewebeteilen in Form von Implantaten oder Transplantaten vorzusehen.

Zur Einstellung der Korrosionsgeschwindigkeit des Werkstoffs ist von Vorteil, wenn die Materialstärke des Werkstoffs in Abhängigkeit von der Zusammensetzung des Werkstoffs so gewählt ist, daß der Abbau- oder Korrosionsvorgang in vivo zwischen 5 Tagen und 6 Monaten, insbesondere zwischen 2 Wochen und 8 Wochen, im wesentlichen abgeschlossen ist.

Hierbei wird erreicht, daß nach einem Anwachsen des Gewebeimplantats die dann nicht mehr benötigte Fixiervorrichtung verschwindet.

Im folgenden werden verschiedene Ausführungsbeispiele der vorliegenden Erfindung gegeben.

### Beispiel 1: Gefäßstütze

Ein erfindungsgemäßer Stent wird aus einem rohrförmigen Grundkörper des metallischen und nachfolgender Bearbeitung gefertigt. Vom mechanischen Aufbau her sind derartige Stents beispielsweise aus der EP 0221570 B1 bekannt, wobei das Material jedoch ein korrosionsbeständiger Edelstahl ist.

Bei dem erfindungsgemäßen Stent nach diesem Beispiel ist das Material eine Legierung mit dem Hauptbestandteil Eisen und den Nebenbestandteilen Chrom und Nickel sowie gegebenenfalls Spuren von anderen Zuschlägen. Die prozentuale Zusammensetzung der Eisenlegierung soll etwa im Bereich 88 - 99% Eisen, 0,5 - 7% Chrom und 0,5 - 3,5% Nickel sowie und unter 5% andere Metalle. Die Wandstärke der Stentstreben soll nach der Bearbeitung zwischen 50 und 100 µm betragen.

In der Praxis wird der erfindungsgemäße Stent in an sich bekannter Weise mit einem Ballonkatheter in ein krankhaft verengtes Blutgefäß eingesetzt und dort dilatiert oder als selbstexpandierender Stent freigesetzt, wobei er das Blutgefäß auf dem gewünschten Durchmesser hält. Eine ohne Stent-Implantation verbleibende Restenose (Recoil) und/oder ein durch die Dilatation induzierter Geweberiß werden wirkungsvoll behandelt. Innerhalb von 2 - 4 Wochen wird der Stent von Intimagewebe überdeckt und behält seine Stützfunktion zunächst bei. Das Blutgefäß erhält durch Gewebewachstum infolge von Eigenreparaturvorgängen im Bereich des implantierten Stents eine neue Eigenstabilität. Das Gefäßlumen wird auf einem optimalen Niveau stabilisiert. Die Wahl des Legierungsmaterials zusammen mit der gewählten Wandstärke führen andererseits dazu, daß der Stent in der Wandung des Blutgefäßes allmählich abgebaut wird und nach etwa 4 - 12 Wochen nur noch in Spuren vorliegt. Die auf Seite 2 geschilderten Nachteile eines Dauerimplantats gehen verloren.

### Beispiel 2: Verschlußsystem

Ein erfindungsgemäßes Verschlußsystem (Schirmchen) wird aus einem metallischen Skelett, an dem ein Kunststoffschirmchen befestigt ist, gefertigt. Derartige Schirmchen sind bekannt beispielsweise aus der Legierung MP35N oder Nitinol. Derartige Verschlußsysteme werden zum Verschluß von Defekten in den Herzscheidewänden verwendet. Die Wandstärke des metallischen Gerüstes beträgt um 500 mm. In der Praxis wird das Schirmchen in an sich bekannter Weise zusammengefaltet und in dem zu verschließenden Defekt freigesetzt. Innerhalb von 3 - 4 Wochen wird das Schirmchen vom körpereigenen Gewebe bedeckt und erhält durch dieses Gewebewachstum eine neue Eigenstabilität. Die Wahl des Legierungsmaterials zusammen mit der Gewebewandstärke führt dazu, daß das metallische Gerüst innerhalb von 4 Wochen bis einigen Monaten abgebaut wird und nach einem Jahr nur noch in Spuren vorliegt. Der Kunststoffanteil des Schirmchens bleibt erhalten, was aufgrund der Flexibilität des Materials unkritisch ist. Der Abbau des metallischen Anteils hat gegenüber den bekannten Schirmchen den Vorteil, daß auch bei unvorhergesehenen Belastungen z.B. bei Verkehrsunfällen keine Gefahr des Durchstoßens von Gefäßwandungen mehr besteht. Dabei wird der erfindungsgemäße Vorteil bereits erreicht, wenn zunächst durch die Degradation eine mechanische Instabilität des Gerüsts entsteht.

### Beispiel 3: Spirale zum Verschließen von Gefäßen (Okkluder)

Eine erfindungsgemäße Spirale (Coil) wird aus einem in Helixform gewickelten metallischen Material gefertigt und die Spirale vorgebogen. Der Durchmesser der Primärwicklung beträgt 0,1 - 1 mm, je nach dem zu verschließenden Gefäß. Derartige Spiralen (Coils) sind bekannt, beispielsweise aus Nitinol, Platinlegierungen oder Wolframlegierungen.

Bei der vorliegenden erfindungsgemäßen Ausführungsform ist das Material eine Legierung mit dem Hauptbestandteil Eisen, den Nebenbestandteilen Nickel und/oder Chrom sowie Spuren von Magnesium und Zink.

In der Praxis wird die Verschlußspirale (Coil) in an sich bekannter Weise in gestrecktem Zustand in einen Herzkatheter eingeführt und durch diesen bis zu dem zu verschließenden Gefäß vorgeschoben. Bei der Freisetzung aus dem Herzkatheter nimmt die Spirale wieder ihre alte Form an und verschließt durch ihr Lumen und ihre Thrombogenität, die durch Dacron oder andere Fasern erhöht werden kann, das zu verschließende Gefäß. Nach Thrombosierung des Gefäßes und Einwachsen von Bindegewebe erhält der Verschlußmechanismus eine neue Eigenstabilität. Die applizierte Spirale wird allmählich abgebaut, so daß nach etwa einem Jahr das implantierte Material nur noch in Spuren vorliegt.

Die insoweit genannten Ausführungsbeispiele lassen sich mit Eisenlegierungen fertigen. Toxische Wirkungen der Materialien bei den zu erwartenden Konzentrationen sind nicht bekannt.

Eisenlegierungen sind vorteilhaft hinsichtlich der mechanischen Stabilität, was sich in den möglichen geringen Wandstärken der Implantate ausdrückt. Das Legierungsmaterial kann deshalb je nach Anwendungsfall ausgewählt werden.

## Patentansprüche

1. Medizinisches Implantat aus einem durch Korrosion in vivo abbaubaren metallischen Werkstoff, **dadurch gekennzeichnet , dass** der Werkstoff Reineisen ist.

2. Medizinisches Implantat aus einem durch Korrosion in vivo abbaubaren metallischen Werkstoff, **dadurch gekennzeichnet , dass** der Werkstoff als Hauptbestandteil Eisen und 0,5% bis 7% Kohlenstoff enthält.

3. Medizinisches Implantat aus einem durch Korrosion in vivo abbaubaren metallischen Werkstoff, **dadurch gekennzeichnet , dass** der Werkstoff als Hauptbestandteil 88 - 99,8% Eisen, 0,1 - 7% Chrom und 0 - 3,5% Nickel sowie unter 5% andere Metalle enthält.

4. Medizinisches Implantat nach einem der vorhergehenden Ansprüche, **dadurch gekennzeichnet , dass** das Implantat eine Gefäßstütze, insbesondere ein Stent ist.

5. Medizinisches Implantat nach einem der vorhergehenden Ansprüche, **dadurch gekennzeichnet , dass** das Implantat eine Befestigungs- oder Stützvorrichtung für die temporäre Fixierung von Gewebeimplantaten oder - transplantaten insbesondere ein Clip ist.

6. Medizinisches Implantat nach einem der vorhergehenden Ansprüche, **dadurch gekennzeichnet , dass** die Materialstärke des Werkstoffs in Abhängigkeit von der Zusammensetzung des Werkstoffs so gewählt ist, dass der Abbau- oder Korrosionsvorgang in vivo im Bereich von 5 Tagen bis zu 6 Monaten, insbesondere zwischen 2 Wochen und 8 Wochen im wesentlichen abgeschlossen ist.

7. Medizinisches Implantat nach einem der vorhergehenden Ansprüche 1 bis 5, **dadurch gekennzeichnet, dass** die Materialstärke des Werkstoffs in Abhängigkeit von der Zusammensetzung des Werkstoffs so gewählt ist, dass der Abbau- oder Korrosionsvorgang in vivo im Bereich von 6 Monaten bis zu 10 Jahren, insbesondere zwischen 1 Jahr und 5 Jahren im wesentlichen abgeschlossen ist.

8. Medizinisches Implantat nach einem der vorhergehenden Ansprüche, **dadurch gekennzeichnet, dass** der Abbau- oder Korrosionsvorgang in vivo zunächst zu einer mechanischen Instabilität führt, bevor der Abbauvorgang im wesentlichen abgeschlossen ist.

## Revendications

1. Implant médical en un matérlau métallique dégradable in vivo par corrosion, **caractérisé en ce que** le matériau est du fer pur.

2. Implant médical en un matériau métallique dégradable in vivo par corrosion, **caractérisé en ce que** le matériau comprend comme composant principal du fer et 0,5 % à 7 % de carbone.

3. Implant médical en un matériau métallique dégradable in vivo par corrosion, **caractérisé en ce que** le matériau comprend comme composant principal 88 à 99,8 % de fer, 0,1 à 7 % de chrome et 0 à 3,5 % de nickel, ainsi que moins de 5 % d'autres métaux,

4. Implant médical selon l'une des revendications précédentes, **caractérisé en ce que** l'implant est un soutien vasculaire, notamment un extenseur.

5. Implant médical selon l'une des revendications précédentes, **caractérisé en ce que** l'implant est un dispositif de fixation ou de soutien destiné à la fixation temporaire d'implants ou greffons tissulaires, notamment un clip.

6. Implant médical selon l'une des revendications précédentes, **caractérisé en ce que** l'épaisseur du matériau est choisie en fonction de la composition du matériau de façon que le processus de dégradation ou de corrosion in vivo soit sensiblement terminé en l'espace de 5 jours à 6 mois, notamment entre 2 semaines et 8 semaines.

7. Implant médical selon l'une des revendications 1 à 5 précédentes, **caractérisé en ce que** l'épaisseur du matériau est choisie en fonction de la composition du matériau de façon que le processus de dégradation ou de corrosion in vivo soit sensiblement terminé en l'espace de 6 mois à 10 ans, notamment entre 1 an et 5 ans.

8. Implant médical selon l'une quelconque des revendications précédentes, **caractérisé en ce que** le processus de dégradation ou de corrosion in vivo aboutit d'abord à une instabilité mécanique avant que ne soit sensiblement terminé le processus de dégradation.

## Claims

1. Medical implant made from a metallic material degradable in vivo through corrosion, **characterized in that** the material is pure iron.

2. Medical implant made from a metallic material degradable in vivo through corrosion, **characterized in that** the material contains iron and 0.5% to 7% carbon as main component.

3. Medical implant made from a metallic material degradable in vivo through corrosion, **characterized in that** the material contains 88 - 99.8% iron, 0.1 - 7% chrome and 0 - 3.5% nickel as well as less than 5% other metals as main component.

4. Medical implant according to any one of the previous claims, **characterized in that** the implant is a tubular support, in particular a stent.

5. Medical implant according to any one of the previous claims, **characterized in that** the implant is a fastening or supporting device for temporary fixing of tissue implants or - transplants, in particular a clip.

6. Medical implant according to any one of the previous claims, **characterized in that** the thickness of the material dependent on the composition of the material is selected in such a way that the degradation- or corrosion in vivo process is fundamentally terminated within a period of 5 days to 6 months, in particular between 2 weeks and 8 weeks.

7. Medical implant according to any one of the previous claims 1 to 5, **characterized in that** the thickness of the material in dependence on the composition of the material is selected in such a way that the degradation- or corrosion in vivo process is fundamentally terminated within a period of 6 months to 10 years, in particular between 1 year and 5 years.

8. Medical implant according to any one of the previous claims, **characterized in that** the degradation- or corrosion in vivo process initially leads to mechanical instability, before the degradation process has fundamentally terminated.
